(19) Europäisches Patentamt
European Patent Office
Office européen des brevets

(11) Veröffentlichungsnummer: **0 094 004**
**B1**

(12) # EUROPÄISCHE PATENTSCHRIFT

(45) Veröffentlichungstag der Patentschrift: **17.04.85**

(51) Int. Cl.[4]: **C 07 C 143/74**, C 07 D 295/12, C 08 G 59/44

(21) Anmeldenummer: **83104345.0**

(22) Anmeldetag: **03.05.83**

(54) **Sulfonsäureamidamine, ihre Herstellung und Verwendung.**

(30) Priorität: **08.05.82 DE 3217372**

(43) Veröffentlichungstag der Anmeldung: **16.11.83 Patentblatt 83/46**

(45) Bekanntmachung des Hinweises auf die Patenterteilung: **17.04.85 Patentblatt 85/16**

(84) Benannte Vertragsstaaten: **CH DE FR GB IT LI NL**

(56) Entgegenhaltungen:
**DE - C - 890 958**
**NL - A - 7 606 239**
**US - A - 3 297 519**
**US - A - 3 458 571**
**US - A - 3 591 556**

(73) Patentinhaber: **BAYER AG, Konzernverwaltung RP Patentabteilung, D-5090 Leverkusen 1 Bayerwerk (DE)**

(72) Erfinder: **Buysch, Hans-Josef, Dr., Brandenburger Strasse 28, D-4150 Krefeld (DE)**
Erfinder: **Wellner, Wolfgang, Dr., Hahnenweg 8, D-5000 Koeln 80 (DE)**
Erfinder: **Gruber, Hermann, Dr., Paul-Klee-Strasse 87, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 094 004 B1

BUNDESDRUCKEREI BERLIN

**Beschreibung**

Die Erfindung betrifft aliphatische, gesättigte Sulfonsäureamidamine, ihre Herstellung aus den entsprechenden Sulfonsäurearylestern durch Umsetzung mit Di- und Polyaminen, die mindestens zwei aliphatische Aminogruppen mit jeweils mindestens einem aktiven Wasserstoffatom enthalten, und die Verwendung der Sulfonamidamine als Modifizierungs- oder Härtungsmittel für Verbindungen, die mindestens eine 1,2-Epoxidgruppe enthalten.

Nach der DE-AS 1 076 366 können aliphatische Polysulfonsäureamide oder aromatische Sulfonsäureamide, die noch primäre, aromatische Aminogruppen enthalten, als Härter für Epoxidharze eingesetzt werden. Diese Stoffe sind jedoch in der Regel feste Produkte und müssen heiß im Epoxidharz gelöst werden. Aber selbst, wenn gute Mischbarkeit gegeben ist, erfolgt eine Durchhärtung der Epoxidharzmassen erst nach mehrstündigem Erhitzen auf 140—160° C.

Gemäß US-PS 2 510 886, Spalte 5, Zeilen 30—34, sollen Sulfonsäureamide, wie p-Toluolsulfonsäureamid oder Naphthalinsulfonsäureamide mit Mischungen aus mehrwertigen Phenolen und Polyepoxidverbindungen in unlösliche und nicht schmelzbare Formkörper übergeführt werden können (vgl. auch US-PS 2 712 001).

Aus der US-PS 3 501 533 ist es bekannt, Polyepoxide mit mehr als einer 1,2-Epoxidgruppe durch Copolymerisate von Vinyl- bzw. Vinylidenmonomeren und Vinylsulfonsäureamiden bei 120° C—230° C zu härten, vorzugsweise in Gegenwart eines Katalysators. Die genannten Copolymerisate sind fest und müssen in der Schmelze oder, gelöst in organischen Lösungsmitteln, mit den Polyepoxiden vermischt werden. Eine Kalthärtung ist praktisch nicht möglich.

Schließlich ist es aus der US-PS 4 122 266 bekannt, unter anderem $C_{35}$—$C_{350}$-Alkansulfonsäureamidamine herzustellen und als Schmieröladditive zu verwenden.

Die Forderungen an Härtungsmittel für Epoxidharze, insbesondere auf dem Lacksektor, gehen immer mehr dahin, nicht nur giftige und teure, sondern überhaupt organische Lösungsmittel zu vermeiden und niedrigviskose Formulierungen anzubieten, die jedoch nur möglich sind, wenn das Härtungsmittel selbst relativ dünnflüssig ist. Darüber hinaus wird auch immer mehr Wert auf variable Systeme gelegt, die sowohl als solche mit Epoxidharzen formulierbar als auch zu mit Wasser verdünnbaren Systemen verarbeitet werden können. Sodann ist es erwünscht, solche Systeme nicht nur als heißhärtende, sondern auch als kalthärtende verfügbar zu haben. Für große Flächen, z. B. im Bausektor, ist Kalthärtung sogar eine unabdingbare Voraussetzung für eine erfolgreiche Anwendung von Epoxidharzsystemen. Für solche Anwendungen stehen aliphatische Amine verschiedener Art zur Verfügung. Diese gestatten zwar eine Kalthärtung, ergeben in der Regel aber Lackfilme mit unansehnlichen, klebrigen, unruhigen und nicht durchgehärteten Oberflächenmassen (Blooming-Effekt). In der Regel sind solche Epoxidharzmassen auch relativ spröde.

Besonders elastische, aber dafür auch weiche Epoxidharzlackierungen erhält man durch Verwendung von Polyamidaminen aus Dimerfettsäuren und Polyalkylen-polyaminen. Diese Härter sind allerdings feste Substanzen, die nur zusammen mit Lösungsmitteln verwendet werden können. Man hat daher in neuerer Zeit zur Vermeidung organischer Lösungsmittel wasserverdünnbare Systeme auf dieser Basis entwickelt. Dies hat jedoch zur Konsequenz, daß solche Systeme nur in verhältnismäßig dünnen Schichten angewendet und auch nicht mit den üblichen festen Zuschlägen und Farbpigmenten gefüllt werden können, weil sonst aus der Masse das zur Verdünnung verwendete Wasser nicht entweichen kann.

Aufgabe der vorliegenden Erfindung war das Auffinden von Härtungs- bzw. Modifizierungsmitteln für Epoxidverbindungen mit mindestens einer 1,2-Epoxidgruppe, die einerseits relativ niedrig-viskos sind, um lösungsmittelfreie, gegebenenfalls pigmentierte und/oder mit Füllstoffen versehene Lack- und Beschichtungsformulierungen zu ermöglichen. Andererseits sollten die Härter-Polyepoxidkompositionen, unabhängig von ihrer Konsistenz, mit Wasser verdünnbar sein. Die Zusammensetzungen sollten auch bei Raumtemperatur schnell durchhärten und Beschichtungen und Lackierungen mit glatter, glänzender, klebfreier Oberfläche und einer hohen Ausgewogenheit zwischen Härte und Elastizität ergeben.

Die Aufgabe wurde dadurch gelöst, daß spezielle aliphatische, gesättigte Sulfonsäureamidamine, wie anspruchsgemäß definiert, als Modifizierungs- und Härtungsmittel eingesetzt werden.

Gegenstand der Erfindung sind somit Sulfonsäureamidamine der allgemeinen Formel I

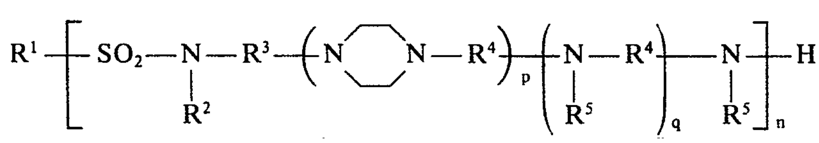 (I)

und deren Gemische, wobei in der Formel I

$R^1$ einen linearen, aliphatischen, gesättigten Kohlenwasserstoffrest mit 10—18 C-Atomen darstellt, der durch ein oder mehrere Chloratome substituiert sein kann,

$R^2$ und $R^5$ gleich oder verschieden, Wasserstoff oder einen Alkylrest mit 1—6 C-Atomen bedeuten,

wobei der Alkylrest eine der Gruppen Hydroxy-, Amino-, Mercapto-, Cyan-, Carboxy- oder Carbamoyl enthalten kann bzw. durch mindestens eine Oxy-(—O—) oder Thiogruppe (—S—) unterbrochen sein kann,

$R^3$ und $R^4$ gleich oder verschieden, einen Alkylenrest mit 2—13 C-Atomen oder einen Cycloalkylenrest mit 5—7 C-Atomen bedeuten und die gleichen Gruppen wie $R^2$ und $R^5$ enthalten können, und

p null oder eine ganze Zahl von 1—3,

q null oder eine ganze Zahl von 1—12 und

n eine ganze Zahl von 1—8 ist.

Vorzugsweise bedeuten $R^1$ einen linearen, aliphatischen, gesättigten Kohlenwasserstoffrest mit 10—18 C-Atomen, $R^2$ und $R^5$ Wasserstoff, $R^3$ und $R^4$ einen Alkylenrest mit 2 oder 3, insbesondere 2 C-Atomen, p = 0 bis 2, insbesondere 0, q eine ganze Zahl von 1—6 und n eine ganze Zahl von 1—5.

Ein wesentlicher, nicht vorhersehbarer Vorteil der erfindungsgemäßen Alkansulfonsäureamidamine gegenüber vergleichbaren Alkancarbonsäureamidaminen liegt darin, daß bei vergleichbarem Gehalt an NH-Äquivalenten die Alkansulfonsäureamidamine flüssig sind, eine erstaunlich niedrige Viskosität aufweisen, gut löslich sind in den üblichen Lacklösemitteln wie Xylol, Butylacetat, Isopropanol, Butanol, Ethylglykolacetat usw. und hervorragend verträglich mit den handelsüblichen Epoxidharzen. Trotz dieser bemerkenswerten Mischbarkeit mit sehr vielen organischen Verbindungen, polaren bis unpolaren, sind die Alkansulfonsäureamidamine in der Regel ausgezeichnet in Wasser löslich.

Solche wäßrigen Lösungen von erfindungsgemäßen Alkansulfonsäureamidaminen vermögen Epoxidharze überraschenderweise ohne Einsatz von Schnellrührern und zusätzlichen Emulgatoren durch einfaches Verrühren mit üblichen Rührern zu emulgieren und erlauben auf diese elegante Weise die Formulierung dünnflüssiger, wasserverdünnbarer, umweltfreundlicher, kalthärtender Epoxidharzsysteme.

Demgegenüber sind Alkancarbonsäureamidamine mit vergleichbarer Struktur und NH-Äquivalenten Verbindungen mit wachsartigem bis hartem, festem Zustand unlöslich in den meisten organischen Lösungsmitteln, zumindest in der Kälte, unlöslich auch in kaltem Wasser und unverträglich mit handelsüblichen Epoxidharzen. Dennoch wurden — allerdings unter praxifernen Bedingungen — mit diesen Alkancarbonsäureamidaminen gehärtete Epoxidharzfilme hergestellt. Solche Filme sind trübe, unansehnlich, klebrig und nicht durchgehärtet.

Die Alkansulfonsäureamidamine weisen ein weiteres unvorhergesehenes Verhalten auf: Die Reaktivität ist innerhalb einer Reihe gegenläufig zur Funktionalität, d. h., die Epoxid-Härter-Gemische haben eine um so höhere Reaktivität je geringer die Anzahl der reaktiven NH-Gruppen innerhalb eines Härtermoleküls ist. Das gerade umgekehrte Verhalten war zu erwarten.

Die Sulfonsäureamidamine gemäß Formel I sind in der Regel Gemische verschiedener Sulfonsäureamidamine. Dies rührt einmal daher, daß bei der Herstellung der Sulfonsäureamidamine aus wirtschaftlichen Gründen nicht die reinen Alkansulfonsäureester mit Polyamin umgesetzt werden, sondern technische Gemische, die neben Alkanmonosulfonsäureestern auch die entsprechenden Di-, Tri-, Tetra- und Pentasulfonsäureester enthalten können. Zum anderen werden häufig nicht nur ein spezielles Polyamin, sondern Mischungen verschiedener Polyamine zur Umsetzung gebracht. Ein besonders bevorzugtes Polyamin ist Pentaethylenhexamin und seine Abmischungen mit Triethylentetramin und/ oder Tetraethylenpentamin und höheren Polyaminen, wie Octaethylennonamin. Die Polyamine können aufgrund ihrer Herstellung (aus Dichlorethan und Ammoniak) auch Piperazinstrukturen in ihrer Molekülkette enthalten.

Die Verbindungen der Formel I können hergestellt werden durch Umsetzung von Sulfonsäurederivaten der allgemeinen Formel II

$$R^1 (SO_2X)_n \qquad (II)$$

in der

$R^1$ und n die für Formel I genannte Bedeutung haben und

X —O-Aryl, vorzugsweise O-Phenyl oder O-Kresyl darstellt,

mit Aminen der allgemeinen Formel III

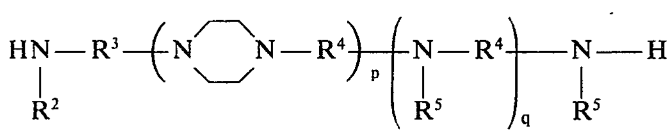

(III)

in der $R^2$, $R^3$, $R^4$, $R^5$, p und q die für Formel I angegebene Bedeutung haben, bei Temperaturen zwischen 80 und 280°C, vorzugsweise 100—250°C, gegebenenfalls in Gegenwart von Katalysatoren, wobei der abgespaltene Rest HX ganz oder teilweise im Reaktionsgemisch verbleiben oder auch entfernt werden kann. Pro Äquivalent Sulfonatester werden vorzugsweise 0,3 bis 3,0 Mol Amin der

Formel III eingesetzt. Überschüssig eingesetztes Polyamin kann im Reaktionsprodukt verbleiben oder durch Destillation entfernt werden.

Die erfindungsgemäßen Sulfonamidamine können vorteilhaft auch in Mischung mit den als Ausgangskomponenten verwendeten Polyaminen zur Härtung von Polyepoxiden eingesetzt werden. In diesem Falle bestehen die Mischungen aus 30 bis 70 Mol-% Sulfonamidamin der Formel I und 70 bis 30 Mol-% der für die Herstellung der Sulfonamidamine eingesetzten Polyamine der Formel III.

Die erfindungsgemäßen Produkte können auch in Mischung mit anderen Epoxidharzhärtern wie Isophorondiamin oder Diamino-dicyclohexylmethan eingesetzt werden.

Die erfindungsgemäßen Sulfonsäureamidamine können auch aus den entsprechenden $C_{10}-C_{18}$-Alkansulfochloriden und den Aminen gemäß Formel III hergestellt werden.

Die als Ausgangsstoffe eingesetzten Sulfonsäureester sind bekannt und können beispielsweise nach dem Verfahren der US-PS 2 683 161 erhalten werden.

Falls gewünscht, können die erfindungsgemäßen Sulfonsäureamidamine der Formel I (1 Mol) auch mit 0,5—2,3 Mol, vorzugsweise 0,8—1,9 Mol, eines gesättigten Lactams, insbesondere $\varepsilon$-Caprolactam und/oder 0,5—2,8 Mol, vorzugsweise 0,6—2,5 Mol, Acrylnitril, Methacrylnitril oder deren Mischungen umgesetzt werden. Auch eine Modifizierung (1 Mol erfindungsgemäßes Sulfonsäureamidamin) mit 0,01—1,8 Mol, vorzugsweise 0,1—1,6 Mol eines 1,2-Alkylenoxids, wie Ethylenoxid, Propylenoxid, Phenylglycidylether, ist möglich. Die vorgenannten Umsetzungen können nach den Verfahren, wie in der US-PS 4 263 162 angegeben, durchgeführt werden.

Die erfindungsgemäßen Härtungsmittel stellen dünnflüssige bis viskose Öle bzw. Harze dar, die mit bis zu 20 Gew.-% eines geeigneten Verdünners oder mit Wasser zu weiteren Herabsetzungen der Viskosität abgemischt werden können. Die Härtungsmittel besitzen Aminäquivalente (= NH-Äquivalent) von 40 bis 300, wobei unter Aminäquivalent der Quotient aus Molgewicht des Produktes und Anzahl der an Amin-Stickstoff gebundenen Wasserstoffatome zu verstehen ist. Ausgeschlossen sind Wasserstoffatome, die an Sulfonylaminogruppen ($-SO_2-NH-$) gebunden sind.

Die erfindungsgemäßen Härtungsmittel können nicht nur wie übliche aliphatische Polyamine bzw. Polyamidamine zur Härtung von Polyepoxiden in der Wärme eingesetzt werden oder in großen Schichtdicken bei Raumtemperatur, sondern auch für die Härtung von Polyepoxiden in dünnen Schichten (Dicke unter 0,5 cm) bei Raumtemperatur, wobei häufig schon nach 6—12 Stunden klebfreie, staubtrockene, klare, glänzende Filme und Beschichtungen mit einwandfreier Oberfläche erhalten werden.

Pro Epoxidäquivalent können 0,6 bis 1,5, vorzugsweise 0,8 bis 1,2 Aminäquivalente (= NH-Äquivalente) der erfindungsgemäßen Härtungsmittel zur Härtung eingesetzt werden.

Unter dem Epoxidäquivalent wird die Menge an 1,2-Epoxidverbindung verstanden, in der eine 1,2-Epoxidgruppe enthalten ist. Entsprechend stellt der Epoxidwert die Anzahl von 1,2-Epoxidgruppen dar, die in 100 g Epoxidgruppen enthalten sind.

Als Epoxide kommen die bekannten, üblichen Polyepoxide mit mehr als einer 1,2-Epoxidgruppe in Frage. Das sind unter anderem Polyglycidylether mehrwertiger Phenole, beispielsweise aus Brenzkatechin, Resorcin, Hydrochinon, aus 4,4'-Dihydroxydiphenylmethan, aus 4,4'-Dihydroxy-3,3'-dimethyldiphenylmethan, aus 4,4'-Dihydroxydiphenyldimethylmethan (Bisphenol A), aus 4,4'-Dihydroxydiphenylmethylmethan, aus 4,4'-Dihydroxydiphenylcyclohexan, aus 4,4'-Dihydroxy-3,3'-dimethyldiphenylpropan, aus 4,4'-Dihydroxydiphenyl, aus 4,4'-Dihydroxydiphenylsulfon, aus Tris-(4-hydroxyphenyl)-methan, aus den Chlorierungs- und Bromierungsprodukten der vorstehend genannten Diphenole, insbesondere aus Bisphenol A; aus Novolaken (d. h. aus Umsetzungsprodukten von ein- oder mehrwertigen Phenolen mit Aldehyden, insbesondere Formaldehyd, in Gegenwart saurer Katalysatoren), aus Diphenolen, die durch Veresterung von 2 Mol des Natriumsalzes einer aromatischen Oxycarbonsäure mit einem Mol eines Dihalogenalkans oder Dihalogendialkylethers erhalten wurden (vgl. britische Patentschrift 1 017 612), aus Polyphenolen, die durch Kondensation von Phenolen und langkettigen, mindestens 2 Halogenatome enthaltenden Halogenparaffinen erhalten wurden (vgl. britische Patentschrift 1 024 288).

Außer den Epoxyharzen auf Basis eines mehrwertigen Phenols und einer Chlorepoxyverbindung können auch die epoxydierten Ringverbindungen gemäß US-Patentschrift 2 716 123 verwendet werden.

Weiter seien genannt Glycidylether mehrwertiger Alkohole, beispielsweise aus 1,4-Butandiol, 1,4-Butendiol, Glycerin, Trimethylolpropan, Pentaerythrit und Polyethylenglykolen.

Von weiterem Interesse sind Triglycidylisocyanurat, N,N'-Diepoxypropyloxamid, Polyglycidylthioether aus mehrwertigen Thiolen, wie beispielsweise aus Bismercaptomethylbenzol, Diglycidyl-trimethylentrisulfon, epoxidiertes Polybutadien, epoxidiertes Leinöl, Vinylcyclohexendiepoxid.

Außerdem kommen in Frage: Glycidylester mehrwertiger aromatischer, aliphatischer und cycloaliphatischer Carbonsäuren, beispielsweise Phthalsäurediglycidylester, Terephthalsäurediglycidylester, Tetrahydrophthalsäurediglycidylester, Adipinsäure-diglycidylester, Hexahydrophthalsäurediglycidylester, die gegebenenfalls durch Methylgruppen substituiert sein können, und Glycidylester von Umsetzungsprodukten aus 1 Mol eines aromatischen oder cycloaliphatischen Dicarbonsäureanhydrids und 1/2 Mol eines Diols bzw. 1/n Mol eines Polyols mit n Hydroxylgruppen, etwa Glycidylcarbonsäureester der allgemeinen Formel

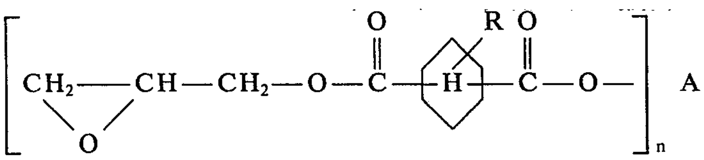

worin A einen mindestens 2wertigen Rest eines gegebenenfalls durch Sauerstoff und/oder cycloaliphatische Ringe unterbrochenen, aliphatischen Kohlenwasserstoffs oder den 2wertigen Rest eines cycloaliphatischen Kohlenwasserstoffs, R Wasserstoff oder Alkylreste mit 1—3 C-Atomen und n eine Zahl zwischen 2 bis 6 bedeuten, oder Mischungen von Glycidylcarbonsäureesters der angegebenen allgemeinen Formel (vgl. britische Patentschrift 1 220 702).

Von Interesse sind außerdem Epoxidharze, die mit Monocarbonsäuren umgesetzt wurden, insbesondere mit Fettsäuren, wie denjenigen aus Leinsamenöl, Sojaöl, Safraöl, Perillaöl, Tungöl, Mohnsamenöl, Sonnenblumenöl, Tallöl, Walnußöl, dehydratisiertes Ricinusöl, Heringöl u. dgl. Die Epoxidharze können in einfacher Weise verestert werden, indem man sie in Gegenwart von einer oder mehreren Carbonsäuren unter Rückflußkühlung erwärmt und das Wasser gleichzeitig azeotrop entfernt.

Bevorzugt sind Polyglycidylether mehrwertiger Phenole und mehrwertiger Alkohole sowie Glycidylester, insbesondere Polyepoxide auf Basis des Bisphenol A.

Die Harzkompositionen aus Härter und Epoxidkomponenten können zusätzlich Extender wie Cumaronöl, Verdünnungsmittel wie Dibutylphthalat enthalten, wenn sie auch vorzugsweise ohne solche eingesetzt werden, ferner Reaktivverdünner wie Monoglycidester oder Monoglycidether wie Umsetzungsprodukte von Phenolen mit Epichlorhydrin, Katalysatoren, die die Härtung beschleunigen, wie Alkohole, Phenole, tert. Amine oder organische Säuren wie Salicylsäure und Amine, Säuren wie $BF_3$ oder dessen Addukte mit Alkoholen, Phosphorverbindungen wie Triphenylphosphit, Retarder, die die Aushärtung verzögern, wie Ketone oder Ester, und schließlich feste Zuschläge, Füllmaterialien und Verstärkungsstoffe wie Talkum, Quarzmehl, Titandioxid, Kieselgur, Schwerspat, Asbest, Glasfasern, Zinkstaub, Glimmer, Sikkative, Tixotropierungsmittel und Farbstoffpigmente wie Eisenoxid, Chromoxid und Cadmiumsulfid. Bei Außenanwendung können auch UV-Stabilisatoren zugesetzt werden. Die Harzkompositionen können sehr vorteilhaft als wäßrige Emulsionen zum Einsatz kommen.

Mit besonderem Vorteil finden die beschriebenen Systeme dort Anwendung, wo kalthärtende Epoxidharze in der Regel eingesetzt werden, z. B. für die Fertigung von Gießkörpern und Harzmatten, besonders aber auf dem Beschichtungs- und Lackierungssektor.

Als zu überziehende bzw. zu beschichtende Substrate kommen Metalle, Holz, Papier, Kartonagen, Textilien, Leder, Glas, Kunststoffe, keramische Materialien, Steine, Beton u. a. in Frage.

Die in den Beispielen angegebenen Teile und Prozente beziehen sich auf das Gewicht, sofern nicht anders vermerkt.

Die in den Beispielen angegebenen Auslaufviskositäten wurden nach DIN 53 211 im 6-mm-Auslaufbecher gemessen.

Das in den Beispielen eingesetzte Epoxidharz I ist ein Bisphenol-A-Diglycidylether mit einem Epoxidwert von 0,55. Epoxidharz II stellt eine Mischung aus Epoxidharz I (70 Gew.-Teile) und 30 Gew.-Teilen tert.-Butylphenylglycidylether dar, wobei das zur Herstellung des tert.-Butylphenylglycidylethers eingestellte tert.-Butylphenol ein technisches Gemisch aus ortho- und para-tert.-Butylphenol ist. Das tert.-Butylphenol wird in bekannter Weise mit Epichlorhydrin in Gegenwart von Natronlauge zu Glycidylether umgesetzt. Der Epoxidwert des Epoxidharzes II ist 0,5.

Die in den Beispielen angegebenen Filmeigenschaften wurden an Filmen gemessen, die aus dem angegebenen Epoxid/Härter-Gemisch nach Auftragen in ca. 0,3 bis 0,5 mm dicker Schicht auf Glasplatten nach 24 Stunden Trocknung bei Raumtemperatur (ca. 23° C) erhalten wurden. Epoxidharz und Härter wurden jeweils im äquivalenten Verhältnis eingesetzt.

Die Filme wurden nach folgenden Gesichtspunkten beurteilt: Klebfreiheit, Glanz, Härte (Kratzfestigkeit), Oberflächenstörungen (= Bloomingeffekt, wellenförmige Störungen, Orangenschaleneffekt) und Durchsichtigkeit.

Erste Voraussetzung für die Eignung eines Epoxidharzes bei Raumtemperatur ist natürlich die Klebfreiheit des Films (Oberfläche), die wie folgt ermittelt wurde:

Die Unterlage wird mit der Lackschicht nach oben auf eine tarierte Waage gelegt, die mit einem Gegengewicht von 1 kg belastet wird. Auf die Lackschicht wird ein kleiner, fettfreier Wattebausch von 2 bis 3 cm Durchmesser gelegt und auf diesen eine kleine Metallscheibe mit einem Durchmesser von 2 cm. Jetzt wird mit dem Finger auf die Scheibe gedrückt, bis die Waage im Gleichgewicht steht, und so wird die Waage während 10 sec im Gleichgewicht gehalten. Nach Entfernen der Metallscheibe wird versucht, den Wattebausch durch sanftes Blasen zu entfernen. Die Lackschicht ist klebfrei, wenn der Bausch nicht mehr an der Lackschicht klebt und auch keine Härchen hinterbleiben.

## Beispiel 1

a) 184 g (ca. 0,5 Mol) n-Pentadecansulfonsäure-phenylester mit ca. 7% n-Pentadecandisulfonsäurediphenylester und 278 g (ca. 1,2 Mol) Pentaethylen-hexamin werden bei ca. 20 mbar und 180—190° C miteinander innerhalb von 2 Stunden umgesetzt, wobei Phenol abdestilliert wird. Man erhält ein hellbraunes Öl mit einer Auslaufviskosität von 3,5 Min. und einem HN-Äquivalent von 48.
Mit diesem Härter und dem Epoxidharz I erhält man einen klaren, glatten, kratzfesten praktisch klebfreien Film.

b) An 365 g des unter a) hergestellten Öles werden 21 g (0,4 Mol) Acrylnitril bei 60°C in 30 Min. addiert. Man erhält ein hellbraunes Öl mit einer Auslaufviskosität von 4 Min. und einem NH-Äquivalent von 58.
Mit diesem Härter liefern Epoxidharze I und II glänzende, ruhige, sehr kratzfeste und klebfreie Lackfilme.

c) 100 g des Produktes aus a) werden bei 50—60°C in 1 h mit 7,5 g (0,05 Mol) Phenylglycidylether umgesetzt. Der ölige Härter (Auslaufzeit 4,5 Min.) mit dem NH-Äquivalent 72 ergibt mit Epoxidharz I klare, klebfreie, kratzfeste und elastische Lackfilme.

## Beispiel 2

Beispiel 1a wird wiederholt, aber mit 155 g (ca. 0,67 Mol) statt 278 g Pentaethylenhexamin. Man erhält ein bräunliches Öl (Auslaufviskosität 4 min; NH-Äquivalent 57), das mit Epoxidharz I einen glatten, klaren, klebfreien und kratzfesten Lackfilm, mit Epoxidharz II eine elastische, verhältnismäßig weiche, durchsichtige Harzplatte mit glatter, klebfreier Oberfläche ergibt.

## Beispiel 3

Ein Gemisch von

75 g Triethylentetramin (0,51 Mol),
48 g Tetraethylenpentamin (0,25 Mol),
40 g Pentaethylenhexamin (0,17 Mol) und
368 g (1 Mol) Sulfonsäureester wie in Beispiel 1a

wird bei 10 mbar und 150—180°C erhitzt und Phenol über eine 50-cm-Vigreux-Kolonne abdestilliert. Das dickflüssige Öl (Auslaufviskosität 7 Min.) mit einem NH-Äquivalent von 99 ergibt mit den Epoxidharzen I und II elastische, durchsichtige Harzplatten und Filme mit klebfreier und glatter Oberfläche.

## Beispiel 4

Beispiel 3 wird wiederholt, wobei aber folgende Aminmengen eingesetzt werden:

97 g (ca. 0,66 Mol) Triethylentetramin,
62 g (ca. 0,33 Mol) Tetraethylenpentamin und
51 g (0,22 Mol) Pentaethylenhexamin.

Das klare hellbraune Öl mit einer Auslaufviskosität von 3 Min. 10 Sek. und einem NH-Äquivalent von 80 liefert mit den Epoxidharzen I und II glatte, klare, klebfreie und kratzfeste Lackfilme und elastische Harzplatten mit glatter und glänzender Oberfläche.

## Beispiel 5

a) Beispiel 1 wird wiederholt, wobei 232 g (1 Mol) Pentaethylenhexamin eingesetzt werden. Das erhaltene Öl hat ein NH-Äquivalent von 53, eine Auslaufzeit von 3,5 Min. und ergibt mit Epoxidharz II kratzfeste, glatte und klebfreie Lackfilme.

b) 165 g des Produktes aus 5a werden bei 60°C mit 13 g Propylenoxid umgesetzt. Das NH-Äquivalent verschiebt sich dadurch auf 61. Die Auslaufzeit beträgt 3,8 Min.

Mit den Epoxidharzen I und II erhält man kratzfeste, klebfreie und glatte Lackfilme.

## Beispiel 6

552 g (1,5 Mol) Sulfonsäureester wie in Beispiel 1a und 522 g (2,25 Mol) Pentaethylenhexamin werden wie in Beispiel 1a kondensiert und 135 g Phenol abdestilliert. Man erhält 932 g eines hellbraunen, klaren Öles, das anschließend mit 74 g (1,4 Mol) Acrylnitril bei 60° C in 30 Min. umgesetzt wird. Das dickflüssige Öl besitzt ein NH-Äquivalent von 88 und eine Auslaufzeit von 4 Minuten.

Mit Epoxidharz I erhält man klare, elastische, klebfreie, glatte und kratzfeste, mit Epoxidharz II ebensolche, aber noch nicht ganz kratzfeste Lackfilme.

## Beispiel 7

a) Ein Gemisch von 900 g des Sulfonsäureesters wie in Beispiel 1a und 1278 g Pentaethylenhexamin werden 5 h bei 150° C unter Stickstoff gerührt und dann bei 60° C mit 537 g Acrylnitril in 40—60 Minuten umgesetzt. Das gelbe Öl mit einem NH-Äquivalent von 98 ergibt mit Epoxidharz I klare, kratzfeste, klebfreie und glatte Lackfilme.

b) Der Härter nach 8a) läßt sich auch in wäßrigen Systemen anwenden. Dazu wird eine Lösung des Härters in 50% seines Gewichtes an Wasser hergestellt und mit Epoxidharz I gründlich vermischt. Die erhaltene Emulsion ergibt klare, kratzfeste und klebfreie Lackfilme. Durch Zugabe einer sehr geringen Lösungsmittelmenge (1—2 Gew.-%, bezogen auf die Gesamtmenge) wie Xylol und n-Butanol wird die Verdunstung des Wassers beschleunigt und der Verlauf noch verbessert, so daß die Filme zudem hochglänzend werden.

## Beispiel 8

586 g (1 Mol) eines Gemisches von n-Tridecan- bis n-Octadecansulfonsäurephenylestern, das im Mittel n-Pentadecansulfonsäurephenylestern entspricht und ca. 26% Mono-, 33% Di-, 24% Tri-, 12% Tetra- und ca. 4—5% Pentasulfonsäureester enthält und 552 g (2,48 Mol) Pentaethylenhexamin werden 7 h unter Stickstoff bei 150—160° C gerührt.

Das erhaltene hochviskose gelbbraune Harz mit einem NH-Äquivalent von 106 wird mit 50% seines Gewichtes an Wasser verdünnt, mit Epoxidharz I zu einer Emulsion verrührt, die nach Ausstreichen zu klaren, kratzfesten klebfreien und glänzenden Lackfilmen härtet.

Das n-Tridecan- bis n-Octadecansulfonsäurephenylestergemisch besteht aus 6% Tridecan-, 27% Tetradecan-, 33% Pentadecan-, 22% Hexadecan-, 10% Heptadecan- und 2% Octadecansulfonsäurephenylestern.

## Beispiel 9

586 g des Sulfonsäureesters wie in Beispiel 8 und 348 g (2,48 Mol) Triethylentetramin werden 7 h bei 150—160° C unter Stickstoff gerührt und dann bei 60° C mit 48 g (0,9 Mol) Acrylnitril umgesetzt. Man nimmt das gelbbraune Harz (NH-Äquivalent 117) in 50% seines Gewichtes an Wasser auf und emulgiert mit Epoxidharz I. Die Emulsion ergibt klare, kratzfeste, klebfreie und glänzende Lackfilme.

## Beispiel 10

586 g des Sulfonsäureesters wie in Beispiel 8 und 450 g (2,48 Mol) Tetraethylenpentamin werden 7 h bei 150—160° C unter Stickstoff gerührt und anschließend bei 60° C mit 104 g (1,95 Mol) Acrylnitril umgesetzt (NH-Äquivalent 112). Lösen des Härters in 30% seines Gewichtes in Wasser und Verrühren mit Epoxidharz I liefert eine Emulsion, die klare, klebfreie, glänzende und kratzfeste Lackierungen ergibt.

## Beispiel 11

a) 150 g (0,65 Mol) des Sulfonsäureesters gemäß Beispiel 9 und 60 g (0,36 Mol) Pentaethylenhexamin werden 1 h auf 160° C erhitzt, dann ist das gelbbraune, dickflüssige Harz wasserlöslich, NH-Äquivalent = 272.
Mit ca. 20% seines Gewichtes an Wasser wird dieser Härter zu einem Öl verrührt. Nach Zugabe der äquivalenten Menge Epoxidharz I erhält man durch bloßes Verrühren ohne Emulgator bzw. mit einem Schnellrührer eine beständige feinteilige Emulsion, die nach Ausstreichen klare, klebfreie und glänzende Lackfilme ergibt.

b) 102 g des unter a) gewonnenen Harzes werden bei 19 mbar bis zu einer Sumpftemperatur von

190° C erhitzt und von Phenol befreit. NH-Äquivalent = 216.
Das hochviskose Harz wird mit der 4fachen Menge Wasser zu einem dünnflüssigen klaren Öl vermischt. Die äquivalente Menge Epoxidharz I läßt sich durch bloßes Verrühren wie unter a) in eine beständige feinteilige Emulsion überführen mit einer Topfzeit von ca. 2—2¹/₂ h. Diese Emulsion liefert klare, glänzende Lackfilme, die schon nach 6—7 h klebfrei ausgehärtet sind.

Beispiel 12

Ein Gemisch von 312 g (1 Mol), bezogen auf Sulfonatgruppe, n-Pentadecansulfonsäurephenylester mit ca. 25% Disulfonat und 232 g (1 Mol) Pentaethylenhexamin wird nach 5stündigem Rühren bei 160° C unter $N_2$ bei 10 mbar bei einer Sumpftemperatur von 190° C von Phenol befreit. Man erhält ein hellbraunes dickflüssiges Öl mit einer Auslaufviskosität von 25 Minuten und einen NH-Äquivalent von 78. Das Produkt ist klar in kaltem Wasser löslich. Mit Epoxidharz I erhält man einen glatten, klaren und kratzfesten Lackfilm.

Beispiel 13

Ein Gemisch von 312 g (1 Mol) n-Pentadecan-sulfonsäurephenylester mit 25% Disulfonsäurephenylester und 182 g (1 Mol) Tetraethylenpentamin wird 5 h unter $N_2$ bei 160° C gerührt und anschließend bis zu einer Sumpftemperatur von 190° von Phenol befreit. Man erhält ein bräunliches Öl mit einer Auslaufviskosität von 22 Minuten NH-Äquivant = 86. Das Produkt ist klar in kaltem Wasser löslich. Mit Epoxidharz I erhält man einen klaren, glänzenden und kratzfesten Lackfilm.

Beispiel 14

Ein Gemisch von 312 g (1 Mol) n-Pentadecan-sulfonsäurephenylester mit ca. 25% Disulfonsäurephenylester und 146 g (1 Mol) Triethylentetramin wird 7 h unter $N_2$ bei 160° C gerührt und dann bei 10 mbar bis zu einer Sumpftemperatur von 190° C von Phenol befreit. Man erhält ein gelbbraunes dickflüssiges Öl mit einer Auslaufviskosität von 18 Minuten und einem NH-Äquivalent von 93. Das Produkt ist in kaltem Wasser klar löslich. Mit Epoxidharz I erhält man einen klaren, glänzenden und kratzfesten Lackfilm.

Beispiel 15

Die Härter aus den Beispielen 12—14 werden hinsichtlich ihrer Aktivität vergleichend geprüft. Dazu vermischt man Härter und Epoxidharz mengenmäßig entsprechend dem NH-Äquivalent rasch miteinander und läßt jeweils 100 g dieser Mischung unter Temperaturkontrolle in einem zylindrischen, isolierenden Gefäß reagieren. Die Aktivität des Härters ist um so größer, je rascher die Temperatur der Mischung ansteigt, d. h. je kürzer die Zeit ist bis zum Erreichen einer bestimmten Temperatur. Der Vergleichbarkeit halber wird die Zeit gemessen, in der die Temperatur von 22° C auf 50° C steigt.
Die folgende Tabelle zeigt nun die Zusammensetzung und Daten der Härter aus den Beispielen 12 bis 14 und dazu die jeweils gemessene Reaktionszeit.

| Bsp. Nr. | Mol SPE*) | Mol PPA*) | Anzahl bas. NH im Molekül Härter | NH-Äquiv. | Teile Epoxid I | Teile Härter | Reaktionszeit von 22—50° C |
|---|---|---|---|---|---|---|---|
| 12 | 1 | 1 Pentaethylenhexamin | 6 | 78 | 70 | 30 | 30 Min. |
| 13 | 1 | 1 Tetraethylenpentamin | 5 | 86 | 68 | 32 | 21 Min. |
| 14 | 1 | 1 Triethylentetramin | 4 | 93 | 66 | 34 | 14 Min. |

*) SPE = Sulfonsäurephenylester
*) PPA = Polyethylenpolyamin

Aus der Tabelle läßt sich ablesen, daß die Reaktionszeit um so kürzer und damit die Aktivität um so höher ist, je höher das NH-Äquivalent liegt, oder je weniger basische NH-Gruppen das Härtermolekül enthält. Dies ist ein völlig unerwarteter Befund.

Für die Vergleichsversuche werden handelsübliche Carbonsäuren verwendet, die den Alkansulfonsäurederivaten strukturell sonst möglichst nahestehen. Diese Carbonsäuren werden mit denselben Polyaminen zu Carbonamidaminen kondensiert, die auch für die Herstellung der Sulfonamidamine verwendet werden.

### Vergleichsversuch 1 (Vergleich zu Beispiel 1)

Ein Gemisch von 236 g (1 Mol bez. COOH-Gruppe) Palmitinsäure ($C_{16}$-Carbonsäure) mit 7 Mol-% Dodecandisäure (analog Sulfonatgemisch in Beispiel 1) und 310 g (1,34 Mol) Pentaethylenhexamin wird unter Stickstoff und Rühren auf 160°C gebracht, wobei die Kondensation unter Wasserabspaltung beginnt. Nach 5—6 h ist eine Sumpftemperatur von 200°C erreicht und die Hauptmenge Wasser abgespalten. Man entwässert noch 4 h bei 10 mbar. Dann ist die berechnete Menge Wasser übergegangen. Man erhält ein in der Hitze gelbliches viskoses Öl, das nach Abkühlen langsam zu einem festen, harten Kuchen erstarrt. NH-Äquivalent = 49. Das Carbonamidamin ist in Wasser nur in der Hitze löslich und unlöslich in Xylol und Ethanol bei Raumtemperatur.

Für den Einsatz zur Härtung wird das Produkt aufgeschmolzen, abgeschreckt, wodurch es kurzzeitig flüssig bleibt, mit Epoxidharz I rasch gemischt und aufgestrichen. Man erhält einen völlig undurchsichtigen leicht abblätternden Film.

### Vergleichsversuch 2 (Vergleich mit Beispiel 1)

Ein Gemisch von 257 g (1 Mol bez. COOH-Gruppe) Palmitinsäure mit 7 Mol-% Dimerfettsäure (hergestellt aus ungesättigten $C_{18}$-Säuren) und 310 g (1,34 Mol) Pentaethylenhexamin wird wie im Vergleichsversuch 1 kondensiert. Man erhält ein in der Hitze gelbliches Öl, das nach Abkühlen langsam zu einem festen, wachsartigen Produkt erstarrt. NH-Äquivalent = 52. Das Carbonamidamin ist in Wasser nur in der Hitze löslich. In Xylol und Ethanol ist das Produkt bei Raumtemperatur unlöslich.

Das Produkt wird für den Einsatz zur Härtung aufgeschmolzen, abgeschreckt, wodurch es für eine kurze Zeit dickflüssig bleibt, rasch mit Epoxidharz I vermischt und aufgestrichen. Man erhält einen trüben, leicht abblätternden Film. Nach Verdünnen der Mischung mit Wasser erhält man eine instabile Emulsion, die nach Aufstreichen einen trüben, klebrigen Film ergibt.

### Vergleichsversuch 3 (Vergleich mit Beispiel 12)

Ein Gemisch von 173 g (0,68 Mol) Palmitinsäure, 53 g (0,225 Mol) Dodecandisäure und 232 g (1 Mol) Pentaethylenhexamin wird unter Stickstoff und Rühren wie in Vergleichsbeispiel 1 unter Wasserabspaltung kondensiert. Man erhält ein in der Hitze viskoses gelbes Öl, das nach Abkühlen zu einem festen Block erstarrt. NH-Äquivalent = 77.

Das Carbonamidamin ist in Wasser nur in der Hitze löslich; unverträglich ist es mit Epoxidharz I und II.

### Vergleichsbeispiel 4 (Vergleich mit Beispiel 12)

Ein Gemisch von 197 g (0,77 Mol) Palmitinsäure, 66 g (0,118 Mol) Dimerfettsäure (aus ungesättigten $C_{18}$-Säuren) und 232 g (1 Mol) Pentaethylenhexamin wird wie im Vergleichsbeispiel 1 unter Wasserabspaltung kondensiert. Man erhält ein in der Hitze gelbes viskoses Öl, das nach Abkühlen zu einem festen Block erstarrt. NH-Äquivalent = 79.

Das Carbonamidamin ist in Wasser auch in der Hitze schwer löslich und völlig unverträglich mit den Epoxidharzen I und II.

### Vergleichsbeispiel 5 (Vergleich mit Beispiel 14)

Ein Gemisch von 170 g (0,665 Mol) Palmitinsäure, 52 g (0,221 Mol) Dodecansäure und 146 g (1 Mol) Triethylentetramin wird wie in Vergleichsbeispiel 1 kondensiert. Man erhält ein in der Hitze viskoses gelbes Öl, das nach Abkühlen zu einem festen Block erstarrt. NH-Äquivalent = 92.

Das Carbonamidamin ist in Wasser auch in der Hitze unlöslich.

Vergleichsbeispiel 6 (Vergleich mit Beispiel 14)

Ein Gemisch von 185 g (0,72 Mol) Palmitinsäure, 62 g (0,11 Mol) Dimerfettsäure und 146 g (1 Mol) Triethylentetramin wird wie in Vergleichsbeispiel 1 umgesetzt. Man erhält ein heiß viskoses gelbes Öl, das nach Abkühlen zu einem festen Block erstarrt. NH-Äquivalent = 91.

Das Carbonamidamin ist in Wasser auch in der Hitze unlöslich.

**Patentansprüche**

1. Sulfonsäureamidamine der allgemeinen Formel I

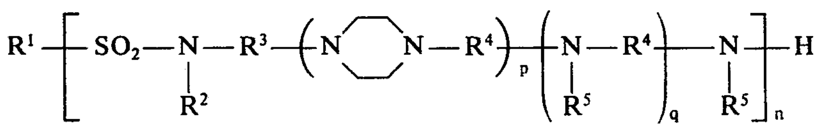

(I)

und deren Gemische, wobei in der Formel I

$R^1$ einen linearen, aliphatischen, gesättigten Kohlenwasserstoffrest mit 10—18 C-Atomen darstellt, der durch ein oder mehrere Chloratome substituiert sein kann,

$R^2$ und $R^5$ gleich oder verschieden, Wasserstoff oder einen Alkylrest mit 1—6 C-Atomen bedeuten, wobei der Alkylrest eine der Gruppen Hydroxy-, Amino-, Mercapto-, Cyan-, Carboxy- oder Carbamoyl enthalten kann bzw. durch mindestens eine Oxy-(—O—) oder Thiogruppe (—S—) unterbrochen sein kann,

$R^3$ und $R^4$ gleich oder verschieden, einen Alkylenrest mit 2—13 C-Atomen oder einen Cycloalkylenrest mit 5—7 C-Atomen bedeuten und die gleichen Gruppen wie $R^2$ und $R^5$ enthalten können und

p null oder eine ganze Zahl von 1—3,

q null oder eine ganze Zahl von 1—12 und

n eine ganze Zahl von 1—8 ist.

2. Sulfonsäureamidamine gemäß Anspruch 1, dadurch gekennzeichnet, daß in Formel I $R^1$ einen linearen, aliphatischen, gesättigten Kohlenwasserstoffrest mit 10—18 C-Atomen, $R^2$ und $R^5$ Wasserstoff, $R^3$ und $R^4$ einen Alkylenrest mit 2 oder 3, p = 0—2, q = eine ganze Zahl von 1—6 und n = eine ganze Zahl von 1—5 bedeuten.

3. Verfahren zur Herstellung von Sulfonsäureamidaminen gemäß Anspruch 1, dadurch gekennzeichnet, daß ein Äquivalent eines Sulfonsäurederivats der allgemeinen Formel II

$$R^1\,(SO_2X)_n \qquad \text{(II)}$$

in der $R^1$ und n die für Formel I genannte Bedeutung haben und X —O-Aryl darstellt, mit 0,3 bis 3,0 Mol an Aminen der allgemeinen Formel III

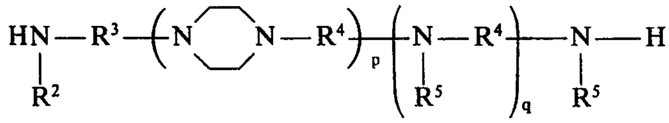

(III)

in der

$R^2$, $R^3$, $R^4$, $R^5$, p und q die für Formel I angegebene Bedeutung haben,

bei Temperaturen zwischen 80 und 280° C, vorzugsweise 100—250° C unter Abspaltung von HX umsetzt.

4. Verwendung der Sulfonamidamine nach Anspruch 1 zur Umsetzung von Epoxidverbindungen mit mindestens einer 1,2-Epoxidgruppe.

**Claims**

1. Sulphonic acid amidamines of the general formula I

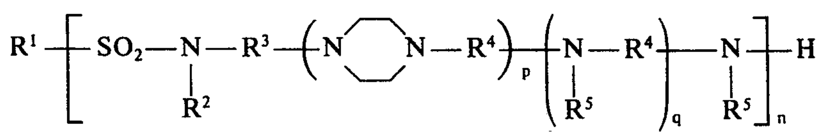

(I)

and mixtures thereof, wherein the formula I

$R^1$ represents a linear, aliphatic, saturated hydrocarbon radical which has 10—18 C atoms and can be substituted by one or more chlorine atoms,
$R^2$ and $R^5$ are identical or different and denote hydrogen or an alkyl radical with 1—6 C atoms, it being possible for the alkyl radical to contain one of the groups: hydroxyl, amino, mercapto, cyano, carboxyl or carbamoyl, or to be interrupted by at least one oxy (—O—) or thio (—S—) group,
$R^3$ and $R^4$ are identical or different, denote an alkylene radical with 2—13 C atoms or a cycloalkylene radical with 5—7 C atoms and can contain the same groups as $R^2$ and $R^5$ and
p is zero or an integer from 1—3,
q is zero or an integer form 1—12 and
n is an integer from 1—8.

2. Sulphonic acid amidamines according to Claim 1, characterised in that in formula I $R^1$ denotes a linear, aliphatic, saturated hydrocarbon radical with 10—18 C atoms, $R^2$ and $R^5$ denote hydrogen, $R^3$ and $R^4$ denote an alkylene radical with 2 or 3, p denotes 0—2, q denotes an integer from 1—6 and n denotes an integer from 1—5.

3. Process for the production of sulphonic acid amidamines according to Claim 1, characterised in that one equivalent of a sulphonic acid derivative of the general formula II

$$R^1 (SO_2X)_n \qquad (II)$$

in which

$R^1$ and n have the meaning mentioned for formula I and
X represents —O-aryl,

is reacted with 0.3 to 3.0 mols of amines of the general formula III

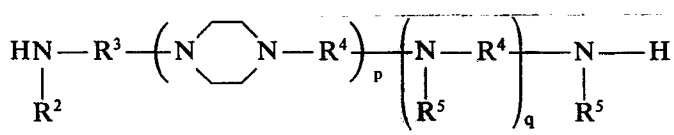

(III)

in which

$R^2$, $R^3$, $R^4$, $R^5$, p and q have the meaning given for formula I,

at temperatures between 80 and 280° C, preferably 100—250° C, with the elimination of HX.

4. Use of the sulphonamidamines according to Claim 1 for the reaction of epoxide compounds containing at least one 1,2-epoxide group.

## Revendications

1. Sulfonamidamines de formule générale I

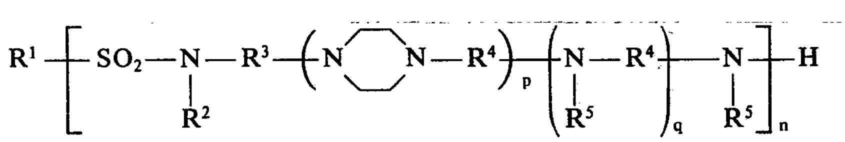

(I)

et leurs mélanges, les symboles de la formule I ayant les significations suivantes:

$R^1$ représente un reste hydrocarboné aliphatique saturé linéaire en $C_1$—$C_{18}$, qui peut être substitué par un ou plusieurs atomes de chlore,
$R^2$ et $R^5$, identiques ou différents, représentent l'hydrogène ou un groupe alkyle en $C_1$—$C_6$, le groupe alkyle pouvant contenir l'un des groupes hydroxy, amino, mercapto, cyano, carboxy ou carbamoyle ou être interrompu par au moins un groupe oxy (—O—) ou thio (—S—),
$R^3$ et $R^4$, identiques ou différents, représentent un reste alkylène en $C_2$—$C_{13}$ ou cycloalkylène en $C_5$—$C_7$ et peuvent contenir les mêmes groupes que $R^2$ et $R^5$, et
p est égal à 0 ou à un nombre entier de 1 à 3,
q est égal à 0 ou à un nombre entier de 1 à 12, et
n est un nombre entier de 1 à 8.

2. Sulfonamidamines selon la revendication 1, caractérisées en ce que, dans la formule I, $R^1$ représente un reste hydrocarboné aliphatique saturé linéaire en $C_{10}-C_{18}$, $R^2$ et $R^5$ représentent l'hydrogène, $R^3$ et $R^4$ un reste alkylène en $C_2-C_3$, $p = 0$ à 2, q est un nombre entier de 1 à 5 et n est un nombre entier de 1 à 5.

3. Procédé de préparation des sulfonamidamines selon la revendication 1, caractérisé en ce que l'on fait réagir un équivalent d'un dérivé d'acide sulfonique de formule générale II

$$R^1 (SO_2X)_n \qquad (II)$$

dans laquelle $R^1$ et n ont les significations indiquées en référence à la formule I et X représente un groupe —O-aryle,
avec 0,3 à 3,0 moles d'amines de formule générale III

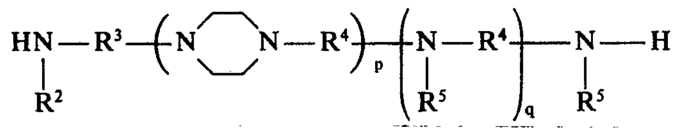

(III)

$R^2$, $R^3$, $R^4$, $R^5$, p et q ayant les significations indiquées en référence à la formule I,
à des températures de 80 à 280° C, de préférence de 100 à 250° C, avec séparation de HX.

4. Utilisation des sulfonamidamines selon la revendication 1 pour la conversion de composés époxydiques portant au moins un groupe 1,2-époxyde.